# EUROPEAN PATENT APPLICATION

(11) **EP 4 338 707 A1**
(43) Date of publication of application: **20.03.2024**
(21) Application number: 21941604.7
(22) Date of filing: 18.09.2021
(51) Int. Cl.: A61F 2/24, A61B 17/00

(54) **CLAMPING INSTRUMENT**

(30) Priority: 11.05.2021 CN 202110512243
(71) Applicant: Shanghai Huihe Healthcare Technology Co., Ltd., Shanghai 201615 (CN)
(72) Inventor: HU, Guanpeng, Shanghai 201615 (CN); XU, Jun, Shanghai 201615 (CN); LIN, Lin, Shanghai 201615 (CN)
(74) Representative: Viering, Jentschura & Partner mbB Patent- und Rechtsanwälte
(86) International application number: PCT/CN2021/119287
(87) International publication number: WO 2022/237037

(57) **Abstract**

The present application provides a clamping instrument, mainly including a locking structure being capable of performing switching action between a locking state and a non-locking state, and a plurality of clamping structures which are connected to the locking structure and capable of being linked with the switching action of the locking structure, so as to adjust each of clamping angles formed by each of the clamping structures, wherein each of the clamping angles formed by each of the clamping structures is adjusted to allow each of the clamping structures to clamp or release each of target tissues; and wherein when the locking structure is in the locking state, the locking structure will apply a continuous pushing force to each of the clamping structures, such that each of the clamping angles formed by each of the clamping structures is maintained to be minimum, thereby clamping each of the target tissues. Therefore, the clamping instrument of the present application provides a stable and reliable clamping force and is suitable for clamping target tissues of different thicknesses.

## Description

The present application claims the priority of the Chinese patent application filed on May 11, 2021 before the CNIPA, China National Intellectual Property Administration with the application number of 202110512243.3 and the title of "Clamping Instrument", which is incorporated herein in its entirety by reference.

### TECHNICAL FIELD

Examples of the present application relate to the technical field of medical instruments, and particularly relate to a clamping instrument.

### BACKGROUND

The common disease of a mitral valve, a tricuspid valve, an aortic valve, a pulmonary valve and the like are valvular insufficiency. Taking the mitral valve as an example, in a systolic phase, a part of blood in a left ventricle flows back to a left atrium through a mitral orifice of insufficiency. The left atrium receives the blood flowing back from the left ventricle and blood delivered from a pulmonary vein simultaneously, and the left atrium has a large increase in blood volume and pressure, leading to hypertrophy of the left atrium.

In a diastolic phase, more blood flows from the left atrium to the left ventricle, resulting in hypertrophy of the left ventricle due to enhanced contraction. After progression from a compensatory phase to a decompensated phase, heart failure occurs in both the left atrium and the left ventricle, followed by pulmonary congestion, pulmonary arterial hypertension, hypertrophy of a right ventricle, hypertrophy of a right atrium, right heart failure, and congestion of systemic circulation.

Conventional treatment means include aggressive surgical methods, or palliative medications against unavoidable heart failure, wherein the surgical methods further include valve replacement and valvuloplasty. In the surgical methods, typical thoracotomy is highly invasive, requires the establishment of extracorporeal circulation, and has a high incidence of complications and a risk of infection. Many patients cannot tolerate the significant surgical risk and can only wait for death.

Current transcatheter products for the treatment of regurgitation of the mitral valve and the tricuspid valve mostly need to change the structure of the heart during a healing process, and thus bring great pressure and inadaptability after heart surgery. Therefore, there is an urgent need for a product which can treat valve regurgitation through a minimally invasive approach without changing the structure of the heart.

### SUMMARY

In view of the above problems, the present application provides a clamping instrument which can provide a reliable clamping force and can be suitable for clamping target tissues of different thicknesses.

The present application provides a clamping instrument, including: a locking structure being capable of performing switching action between a locking state and a non-locking state; and a plurality of clamping structures, each of the clamping structures being respectively connected to the locking structure and capable of being linked with the switching action of the locking structure, so as to adjust each of clamping angles formed by each of the clamping structures; wherein each of the clamping angles formed by each of the clamping structures is adjusted to allow each of the clamping structures to clamp or release each of target tissues; and wherein when the locking structure is in the locking state, the locking structure can apply a continuous pushing force to each of the clamping structures, such that each of the clamping angles formed by each of the clamping structures is maintained to be minimum, thereby clamping each of the target tissues.

Optionally, each of the clamping structures is respectively used for clamping target tissues; and the target tissues include cardiac valves.

Optionally, the clamping instrument further includes a mandrel, the locking structure includes a clamping base and an adjusting base which are coaxially disposed on the mandrel, and each of the clamping structures is respectively connected to the clamping base and the adjusting base; and wherein when the locking structure is gradually switched from the non-locking state to the locking state, a spacing distance between the clamping base and the adjusting base is gradually decreased, and a pivoting angle between each of the clamping structures and the mandrel is gradually decreased.

Optionally, the clamping base is movably disposed on the mandrel and capable of reciprocating in an axial direction of the mandrel; and the adjusting base is fixedly disposed on the mandrel.

Optionally, the locking structure further includes a first fastener and a second fastener which are respectively disposed on the clamping base and the adjusting base; wherein the first fastener and the second fastener can be buckled with each other to maintain the locking structure to be in the locking state; and wherein after the first fastener and the second fastener are buckled with each other, at least a portion of the mandrel can be retained in the clamping instrument, or the mandrel can be completely withdrawn from the clamping instrument.

Optionally, each of the clamping structures respectively includes a main clamping arm, an auxiliary clamping arm, and an elastic arm; the main clamping arm and the auxiliary clamping arm are pivotally connected to the clamping base respectively, and two opposite ends of the elastic arm are pivotally connected to the adjusting base and the main clamping arm respectively; wherein when the locking structure performs the switching action between the locking state and the non-locking state, the spacing distance between the clamping base and the adjusting base can be increased or decreased, so as to drive each of the main clamping arms to pivot relative to the clamping base via the elastic arm; and when the locking structure is maintained to be in the locking state, a continuous elastic pushing force can be applied to each of the main clamping arms via each of the elastic arms to urge each of the main clamping arms to pivot towards a direction close to the auxiliary clamping arm relative to the clamping base, such that a clamping angle formed between the main clamping arm and the auxiliary clamping arm is maintained to be minimum.

Optionally, when the locking structure is in the locking state, the main clamping arm can apply an opposite acting force to the elastic arm according to a thickness of the target tissue, so as to elastically deform the elastic arm to different degrees until an elastic pushing force of the elastic arm is balanced with the opposite acting force of the main clamping arm.

Optionally, the elastic arm includes at least one of an arcuate bent part, an S-shaped bent part, and a Z-shaped bent part.

Optionally, each of the main clamping arms and/or the auxiliary clamping arms is enable to be coaxially or non-coaxially pivoted to the clamping base.

Optionally, the auxiliary clamping arm can be elastically deformed with a stress being applied, so as to pivot towards a direction away from the main clamping arm relative to the clamping base, such that the clamping angle formed between the auxiliary clamping arm and the main clamping arm is increased; and the auxiliary clamping arm can be elastically recovered without a stress being applied, so as to pivot towards a direction close to the main clamping arm relative to the clamping base, such that the clamping angle formed between the auxiliary clamping arm and the main clamping arm is decreased.

Optionally, the auxiliary clamping arm includes a clamping part and a pivoting part, and the clamping part can be pivoted to the mandrel or the clamping base via the pivoting part; and the pivoting part can be elastically deformed with a stress being applied and elastically recovered without a stress being applied, so as to allow the clamping part to pivot relative to the clamping base.

Optionally, each of the auxiliary clamping arms is of an integrated structure or a separate structure.

It can be seen from the above technical solution that the clamping instrument of the examples of the present application applies a continuous pushing force to the clamping structures by means of the locking structure in the locking state, such that the clamping angle formed by each of the clamping structures is maintained to be minimum, thereby allowing the clamping structures to maintain reliable and stable clamping forces.

Furthermore, for the clamping instrument of the examples of the present application, when the locking structure is in the locking state, the main clamping arm of the clamping structure can apply the opposite acting force to the elastic arm according to the thickness of the target tissue, so as to elastically deform the elastic arm to different degrees until the elastic pushing force of the elastic arm is balanced with the opposite acting force of the main clamping arm, thereby allowing the clamping structures to be suitable for clamping the target tissues of different thicknesses by means of this structural design.

### BRIEF DESCRIPTION OF THE DRAWINGS

In order to explain the technical solutions in examples of the present application or in the prior art more clearly, a brief description of the accompanying drawings which are used in the description of the examples or the prior art will be given below. It is obvious that the accompanying drawings described below are only some examples described in the examples of the present application, and for a person of ordinary skill in the art, other accompanying drawings can be obtained according to these accompanying drawings.
Fig. 1A to Fig. 1B are schematic diagrams of three-dimensional structures of different examples of a clamping instrument of the present application.
Fig. 2 is a schematic diagram of a breakdown structure of the clamping instrument of the present application.
Fig. 3 and Fig. 4 are schematic structural diagrams of a locking structure of the clamping instrument of the present application in a non-locking state and in a locking state, respectively.
Fig. 5 and Fig. 6 are side cross-sectional diagrams of the locking structure of the clamping instrument of the present application in the non-locking state and in the locking state, respectively.
Figs. 7 and 8 are partial schematic diagrams of an adjusting base and a clamping base of the present application, respectively.
Fig. 9 and Fig. 10 are partial schematic diagrams of an auxiliary clamping arm of the present application, respectively.
Fig. 11 and Fig. 12 are schematic diagrams of different examples of the clamping instrument of the present application, respectively.
Fig. 13 to Fig. 17 are schematic diagrams of examples of executing cardiac valve prosthetic surgeries by utilizing the clamping instrument of the present application.

### Reference numerals

10: clamping instrument; 20: mandrel; 22: connecting interface; 30: locking structure; 32: clamping base; 322: pivoting shaft; 324: penetrating groove; 34: adjusting base; 342: pivoting hole; 36: first fastener; 38: second fastener; 40: clamping structure; 42: main clamping arm; 422: top end pivoting hole; 424: middle section pivoting hole; 44: elastic arm; 442: first pivoting hole; 444: second pivoting hole; 46: auxiliary clamping arm; 462: clamping part; 464: pivoting part; 466: penetrating part; 52: pivoting shaft; 54: pivoting shaft; 60: target tissue; 70: delivery system; and 80: pulling wire.

### DETAILED DESCRIPTION OF THE INVENTION

In order that those skilled in the art may better understand the technical solutions in examples of the present application, a clear and complete description of the technical solutions in the examples of the present application is provided below in conjunction with the accompanying drawings in the examples of the present application. It is obvious that the examples described are only some, but not all of the examples of the present application. Based on the examples in the embodiments of the present application, all other examples obtained by a person of ordinary skill in the art should fall within the protection scope of the examples of the present application.

Specific implementations of the examples of the present application will be further described below in conjunction with the accompanying drawings in the examples of the present application.

A clamping instrument 10 of this example can be used as an edge-to-edge prosthetic implant in the field of structural heart disease interventional treatment, and can reach a designated anatomical location through a minimally invasive transvascular approach or transauricular approach to achieve prosthetic treatment of cardiac valves.

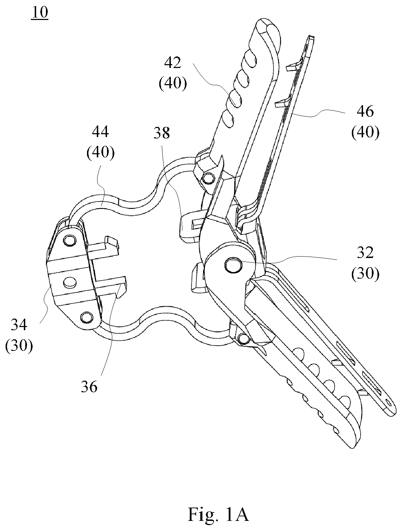

As shown in Fig. 1A to Fig. 6, the clamping instrument 10 of the present application mainly includes a locking structure 30, and a plurality of clamping structures 40.

The locking structure 30 can perform switching action between a locking state and a non-locking state.

Each of the clamping structures 40 is connected to the locking structure 30 and capable of being linked with the switching action of the locking structure, so as to adjust each of clamping angles formed by each of the clamping structures 40.

In this example, each of the clamping angles formed by each of the clamping structures 40 can be adjusted to allow each of the clamping structures 40 to clamp or release each of target tissues.

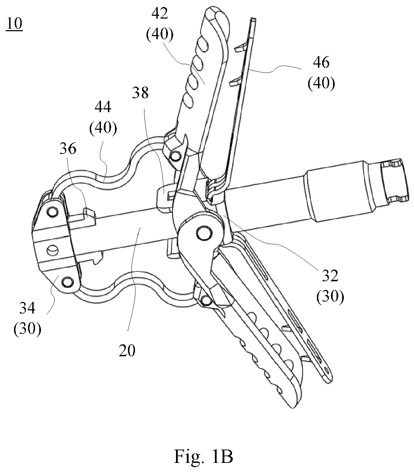

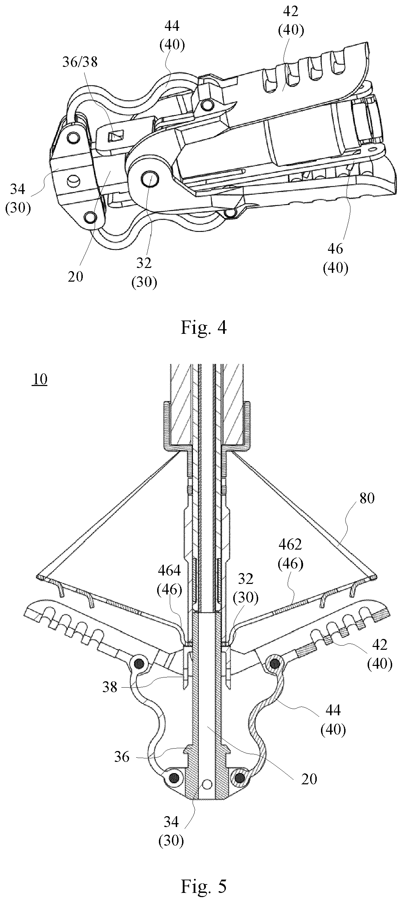

In this example, when the locking structure 30 is switched from the non-locking state to the locking state (namely switched from the state shown in Fig. 1 to the state shown in Fig. 4), a clamping angle formed by each of the clamping structures 40 is gradually decreased.

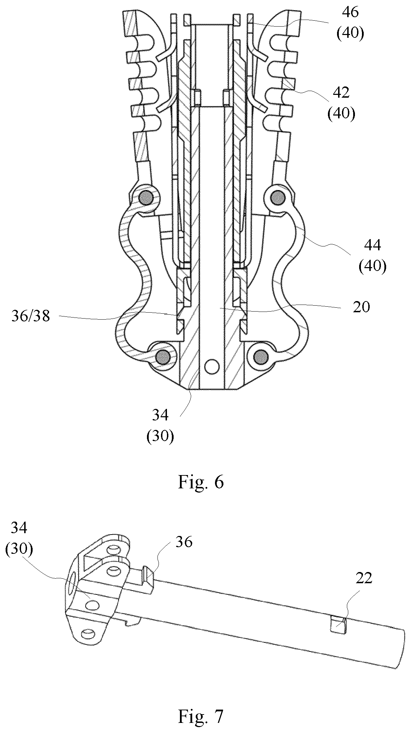

In this example, when the locking structure 30 is in the locking state, the locking structure 30 can apply a continuous pushing force to each of the clamping structures 40, such that each of the clamping angles formed by each of the clamping structures 40 is maintained to be minimum, thereby allowing each of the clamping structures 40 to maintain a stable clamping force, so as to clamp each of the target tissues (namely the states shown in Fig. 4 and Fig. 6).

Optionally, the target tissues are, for example, cardiac valves, including, but not limited to, a mitral valve, a tricuspid valve and the like.

Optionally, the clamping instrument 10 can further include a mandrel 20 (referring to Fig. 1B), and the locking structure 30 can include a clamping base 32 and an adjusting base 34 which are coaxially disposed on the mandrel 20, wherein each of the clamping structures 40 is respectively connected to the clamping base 32 and the adjusting base 34.

Specifically, when the locking structure 30 is gradually switched from the non-locking state to the locking state, a spacing distance between the clamping base 32 and the adjusting base 34 is gradually decreased, such that a pivoting angle between each of the clamping structures 40 and the mandrel 20 is gradually decreased, that is, a free end of the clamping structure 40 moves towards a direction close to the mandrel 20 (referring to the state shown in Fig. 6); on the contrary, when the locking structure 30 is gradually switched from the locking state to the non-locking state, the spacing distance between the clamping base 32 and the adjusting base 34 is gradually increased, such that the pivoting angle between each of the clamping structures 40 and the mandrel 20 is gradually increased, that is, the free end of the clamping structure 40 moves towards a direction away from the mandrel 20 (referring to the state shown in Fig. 5)

Optionally, the clamping base 32 is movably disposed on the mandrel 20 (for example, movably penetrates through the mandrel 20), the adjusting base 34 can be fixedly disposed at a tail end of the mandrel 20, the spacing distance between the clamping base 32 and the adjusting base 34 can be adjusted by means of reciprocating movement of the clamping base 32 in an axial direction of the mandrel 20, thereby driving the clamping structure 40 to pivot relative to the mandrel 20.

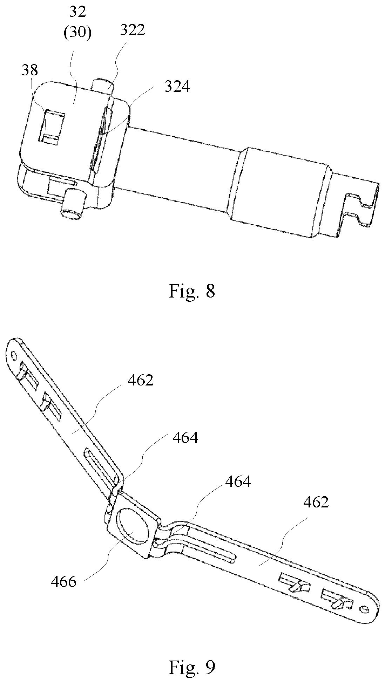

Optionally, the locking structure 30 can further include a first fastener 36 and a second fastener 38 which are respectively disposed on the clamping base 32 and the adjusting base 34 (referring to Fig. 7 and Fig. 8).

In this example, the first fastener 36 and the second fastener 38 can be buckled with each other to maintain the locking structure 30 to be in the locking state.

In this example, the first fastener 36 can include two clamping hooks disposed on two opposite sides of the adjusting base 34, and the second fastener 38 can include two clamping grooves formed in two opposite sides of the clamping base 32. However, this is not a limitation, the first fastener 36 and the second fastener 38 can also be implemented by adopting other buckling structures, and this is not limited in the present application.

In this example, the mandrel 20 has a removable structural design, and is mainly used for providing axial positioning between the clamping base 32 and the adjusting base 34 for positional alignment and buckling operation between the first fastener 36 and the second fastener 38 in a process of clamping the target tissues by the clamping instrument 10.

In this example, after the first fastener 36 and the second fastener 38 are buckled with each other, the mandrel 20 can be selected to be completely withdrawn from the clamping instrument 10 (referring to the state shown in Fig. 1A), or the mandrel 20 can be selected to be completely retained in the clamping instrument 10 to further improve the clamping stability of the clamping instrument 10 (referring to the state shown in Fig. 1B) according to actual use requirements.

In an example, the mandrel 20 can be designed to be a constituent part fixedly disposed in the clamping instrument 10.

In another example, a portion of a delivery system can also be utilized as the mandrel 20 of the clamping instrument 10 to assist the clamping instrument 10 in executing the clamping operation of the target tissues, and after the clamping operation of the target tissues is completed, the mandrel 20 can be separated from the clamping instrument 10 and removed together with the delivery system.

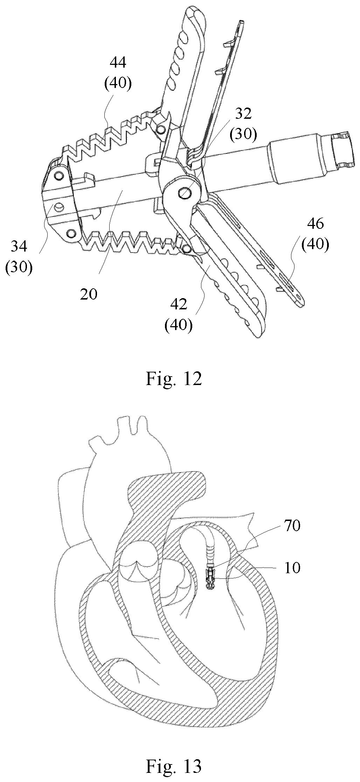

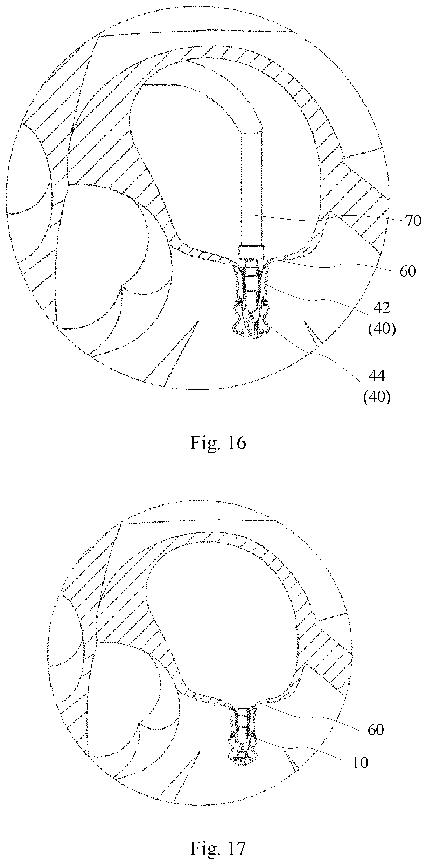

In this example, when the mandrel 20 is designed to be the constituent part fixedly disposed in the clamping instrument 10, a connecting interface 22 can also be designed on the mandrel 20 to allow the clamping instrument 10 to be detachably connected to the delivery system (referring to the detailed description below regarding Fig. 13 to Fig. 17).

Optionally, each of the clamping structures 40 respectively includes a main clamping arm 42, an elastic arm 44, and an auxiliary clamping arm 46.

In this example, each of the main clamping arms 42 and each of the auxiliary clamping arms 46 can be pivotally connected to the clamping base 32, and two opposite ends of each of the elastic arms 44 are pivotally connected to the adjusting base 34 and the main clamping arm 42, respectively.

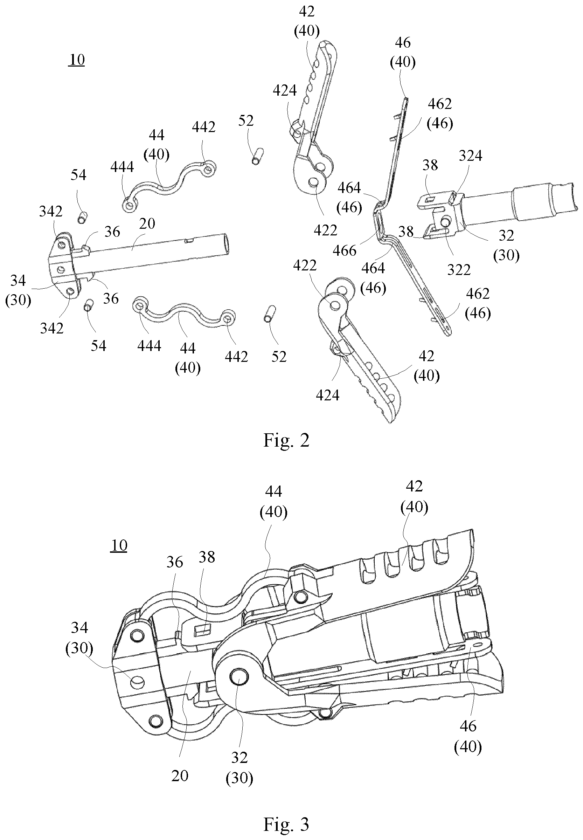

With reference to Fig. 2, in this example, a top end pivoting hole 422 of each of the main clamping arms 42 is pivotally connected to a pivoting shaft 322 of the clamping base 32, the two opposite ends of each of the elastic arms 44 are respectively provided with a first pivoting hole 442 and a second pivoting hole 444, wherein the first pivoting hole 442 of the elastic arm 44 is pivotally connected to a middle section pivoting hole 424 of the main clamping arm 42 via a pivoting shaft 52, and the second pivoting hole 444 of the elastic arm 44 is pivotally connected to a pivoting hole 342 of the adjusting base 34 via a pivoting shaft 54.

In this example, when the locking structure 30 performs the switching action between the locking state and the non-locking state, the spacing distance between the clamping base 32 and the adjusting base 34 which are coaxially disposed on the mandrel 20 can be increased or decreased, wherein when the spacing distance between the clamping base 32 and the adjusting base 34 is decreased, each of the main clamping arms 42 can be driven to pivot relative to the clamping base 32 via the elastic arm 44, such that each of pivoting angles between each of the main clamping arms 42 and the mandrel 20 is gradually decreased (referring to the state shown in Fig. 6); on the contrary, when the spacing distance between the clamping base 32 and the adjusting base 34 is gradually increased, each of the main clamping arms 42 can be driven to pivot relative to the clamping base 32 via the elastic arm 44, such that each of pivoting angles between each of the main clamping arms 42 and the mandrel 20 is gradually increased (referring to the state shown in Fig. 5).

Furthermore, when the locking structure 30 is maintained to be in the locking state, a continuous elastic pushing force can be applied to each of the main clamping arms 42 via each of the elastic arms 44 to urge each of the main clamping arms 42 to pivot towards a direction close to the auxiliary clamping arm 46 relative to the clamping base 32, such that a clamping angle formed between the main clamping arm 42 and the auxiliary clamping arm 46 is maintained to be minimum, and by means of this structural design, the clamping instrument 10 of the present application can provide a long-lasting stable and reliable clamping force, thereby effectively improving the success rate of cardiac valve prosthetic surgeries.

In addition, when the locking structure 30 is in the locking state, the main clamping arm 42 can also apply an opposite acting force to the elastic arm 44 according to a thickness of the target tissue clamped by the main clamping arm 42, so as to elastically deform the elastic arm 44 to different degrees until an elastic pushing force of the elastic arm 44 is balanced with the opposite acting force of the main clamping arm 42.

That is to say, when the locking structure 30 is in the locking state, the main clamping arm 42 and the elastic arm 44 can be adaptively adjusted according to the actual thickness of the target tissue currently clamped, such that different minimum pivoting angles are formed between each of the main clamping arms 42 and the mandrel 20, and this design can not only provide that the clamping instrument 10 of the present application is suitable for clamping the target tissues of different thicknesses, but also ensure that the target tissues of various thicknesses can be effectively clamped, thereby further improving the success rate of the cardiac valve prosthetic surgeries.

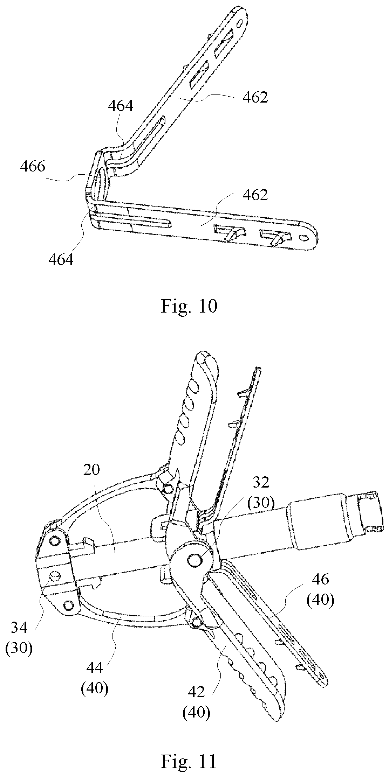

In this example, the elastic arm 44 includes an S-shaped bent part (referring to Fig. 1 to Fig. 6), an arcuate bent part (referring to Fig. 11), or a Z-shaped bent part (referring to Fig. 12).

In other examples, the above elastic arm 44 can also be implemented by adopting spring structures (not shown).

Optionally, the elastic arm 44 can be made of a nickel-titanium alloy material, but this is not a limitation and the elastic arm 44 can also be made of other elastic materials, and it is not limiting in the present application.

Optionally, each of the main clamping arms 42 of each of the clamping structures 40 can be connected to the clamping base 32 in coaxial or non-coaxial pivoting way.

In this example, the auxiliary clamping arm 46 and the main clamping arm 42 can independently pivot relative to the clamping base 32 respectively to adjust the clamping angle formed between the auxiliary clamping arm 46 and the main clamping arm 42, so as to clamp or release the target tissues (for example, cardiac valves).

Optionally, the auxiliary clamping arm 46 can be elastically deformed with a stress being applied, so as to pivot towards a direction away from the main clamping arm 42 relative to the clamping base 32, such that the clamping angle formed between the auxiliary clamping arm 46 and the main clamping arm 42 is increased; and the auxiliary clamping arm 46 can also be elastically recovered without a stress being applied, so as to pivot towards a direction close to the main clamping arm 42 relative to the clamping base 32, such that the clamping angle formed between the auxiliary clamping arm 46 and the main clamping arm 42 is decreased.

Optionally, the auxiliary clamping arm 46 can include a clamping part 462 and a pivoting part 464, wherein the clamping part 462 can be pivoted to the clamping base 32 via the pivoting part 464.

Optionally, each of the auxiliary clamping arms 46 of each of the clamping structures 40 can be designed to be of an integrated structure or a separate structure.

For example, in the examples shown in Fig. 2, Fig. 9 and Fig. 10, each of the auxiliary clamping arms 46 of each of the clamping structures 40 is of an integrated design structure, each of the auxiliary clamping arms 46 can be disposed on the mandrel 20 in a penetrating way through the penetrating part 466, and each of clamping parts 462 of each of the auxiliary clamping arms 46 can extend outwards via each of penetrating grooves 324 of the clamping base 32 and pivot relative to the clamping base 32 with each of the penetrating grooves 324 as a rotating axis (referring to Fig. 2 and Fig. 8).

It should be noted that the way of disposing the auxiliary clamping arm 46 is not limited to the way shown in each of the accompanying drawings of the present application, and a person skilled in the art would be able to adopt any other equivalent design solutions under the technical inspiration of the present application, and it is not limiting in the present application.

In this example, the pivoting part 464 can be elastically deformed with a stress being applied to allow the clamping part 462 to pivot towards a direction away from the main clamping arm 42 relative to the clamping base 32, such that the clamping angle between the main clamping arm 42 and the auxiliary clamping arm 46 (the clamping part 462) is increased; or the pivoting part 464 can also be elastically recovered without a stress being applied to allow the clamping part 462 to pivot towards a direction close to the main clamping arm 42 relative to the clamping base 32, such that the clamping angle between the main clamping arm 42 and the auxiliary clamping arm 46 (the clamping part 462) is decreased, so as to clamp the target tissues.

Referring to Fig. 5 and Fig. 6 in combination, in this example, the free end of the auxiliary clamping arm 46 can be connected to a driving part (for example, a pulling wire 80), and by pulling the free end of the auxiliary clamping arm 46 via the driving part, the pivoting part 464 of the auxiliary clamping arm 46 can be elastically deformed (referring to Fig. 10) to allow the clamping part 462 to pivot relative to the clamping base 32, such that the clamping angle between the auxiliary clamping arm 46 and the main clamping arm 42 is increased; or by releasing the driving part, the pivoting part 464 of the auxiliary clamping arm 46 can be elastically recovered (referring to Fig. 9) to allow the clamping part 462 to pivot relative to the clamping base 32, such that the clamping angle between the auxiliary clamping arm 46 and the main clamping arm 42 is decreased.

A use method of executing cardiac valve repair treatment by utilizing the clamping instrument 10 of the present application will be described below by way of examples with reference to Fig. 13 to Fig. 17.

The clamping instrument 10 is delivered to the vicinity of the target tissue 60 (for example, a cardiac valve) to be repaired by a delivery system 70 (referring to Fig. 13).

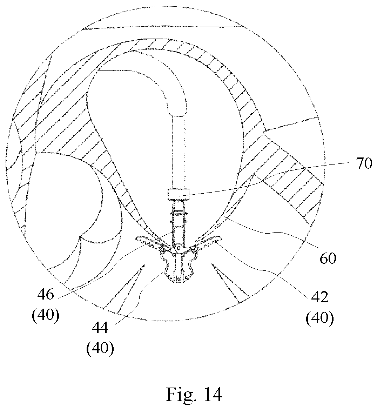

The adjusting base 34 of the clamping instrument 10 is controlled by the delivery system 70 to move towards a distal direction relative to the clamping base 32, such that the spacing distance between the adjusting base 34 and the clamping base 32 is increased, and then the main clamping arm 42 is driven to pivot relative to the clamping base 32 to be opened via the elastic arm 44, that is, the pivoting angle between the main clamping arm 42 and the mandrel 20 is increased, and a pulling acting force is applied to the auxiliary clamping arm 46 by, for example, a pulling wire 80 connected to the auxiliary clamping arm 46, so as to maintain the auxiliary clamping arm 46 to be clung to two opposite sides of the mandrel 20, such that the clamping angle between the main clamping arm 42 and the auxiliary clamping arm 46 is maintained to be large enough to provide for positioning of the target tissue 60 (for example, the cardiac valve) to be repaired between the main clamping arm 42 and the auxiliary clamping arm 46 (referring to Fig. 14).

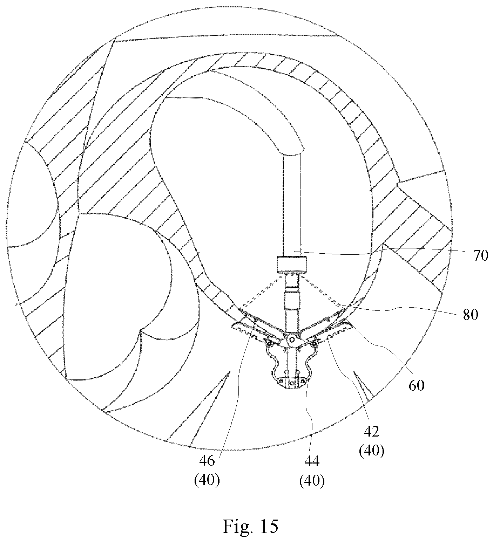

The target tissue 60 (for example, the cardiac valve) is clamped between the main clamping arm 42 and the auxiliary clamping arm 46 by releasing the pulling wire 80 to cause the auxiliary clamping arm 46 to pivot towards a direction close to the main clamping arm 42 relative to the clamping base 32 under the action of an elastic recovering force (referring to Fig. 15).

It should be noted that during this operation, if a clamping position of the target tissue 60 is not ideal, the auxiliary clamping arm 46 can be pulled again by the pulling wire 80 to release the target tissue 60 clamped between the auxiliary clamping arm 46 and the main clamping arm 42, and the target tissue 60 can be recaptured until a clamping state of the target tissue 60 meets the expectation.

The adjusting base 34 of the clamping instrument 10 is controlled by the delivery system 70 to move towards a proximal direction relative to the clamping base 32, such that the spacing distance between the adjusting base 34 and the clamping base 32 is decreased, and then the main clamping arm 42 is driven to pivot in a reverse direction relative to the clamping base 32 via the elastic arm 44, such that the pivoting angle between the main clamping arm 42 and the mandrel 20 is gradually decreased, and the auxiliary clamping arm 46 is driven to be gradually close to the direction of the mandrel 20 together until the adjusting base 34 and the clamping base 32 are buckled with each other, that is, the locking structure 30 is in the locking state.

Ideally, when the locking structure 30 is in this locking state, the main clamping arm 42 will apply a pushing force to the auxiliary clamping arm 46 to cause the auxiliary clamping arm 46 to tightly abut against the clamping base 32, meanwhile, the main clamping arm 42 can also apply an opposite acting force to the elastic arm 44 according to different thicknesses of the target tissues 60 clamped, so as to elastically deform the elastic arm 44 to different degrees until an elastic pushing force applied to the main clamping arm 42 by the elastic arm 44 is balanced with the opposite acting force applied to the elastic arm 44 by the main clamping arm 42, thereby ensuring that the target tissues 60 can be stably and firmly clamped between the main clamping arm 42 and the auxiliary clamping arm 46.

The delivery system 70 is disengaged from the clamping instrument 10 to withdraw the delivery system 70 while only retaining the clamping instrument 10 in the body of a patient, so as to complete the clamping operation of the target tissues 60 (for example, cardiac valves).

In summary, the clamping instrument provided by the present application applies a continuous pushing force to the clamping structures by means of the locking structure in the locking state, so as to maintain the clamping angle formed by each of the clamping structures to be minimum, such that the clamping structures can maintain a stable and reliable clamping force, so as to enhance the clamping effect and improve the success rate of treatment surgeries of mitral valve and tricuspid valve regurgitation.

Furthermore, the main clamping arm of the clamping structure can also apply an opposite acting force to the elastic arm according to the actual thickness of the target tissue clamped by the main clamping arm, so as to elastically deform the elastic arm to different degrees until the elastic pushing force of the elastic arm is balanced with the opposite acting force of the main clamping arm, so as to be suitable for clamping the target tissues of different thicknesses.

Finally, it should be noted that the above examples are merely illustrative of the technical solutions of the examples of the present application, rather than limiting same; although the present application has been described in detail with reference to the foregoing examples, those ordinarily skilled in the art will appreciate that the technical solutions disclosed in the above examples can still be modified, or some of the technical features thereof can be substituted by equivalents; however, these modifications or substitutions do not depart from the spirit and scope of the technical solutions of the examples of the present application in nature.

## Claims

1. A clamping instrument (10), **characterized in that**, comprising:
a locking structure (30) being capable of performing switching action between a locking state and a non-locking state; and
a plurality of clamping structures (40), each of the clamping structures (40) being respectively connected to the locking structure (30) and capable of being linked with the switching action of the locking structure (30), so as to adjust each of clamping angles formed by each of the clamping structures (40);
wherein each of the clamping angles formed by each of the clamping structures (40) is adjusted to allow each of the clamping structures (40) to clamp or release each of target tissues; and
wherein when the locking structure (30) is in the locking state, the locking structure (30) will apply a continuous pushing force to each of the clamping structures (40), such that each of the clamping angles formed by each of the clamping structures (40) is maintained to be minimum, thereby clamping each of the target tissues.

2. The clamping instrument according to claim 1, wherein the target tissues comprise cardiac valves.

3. The clamping instrument according to claim 2, further comprising a mandrel (20), wherein the locking structure (30) comprises a clamping base (32) and an adjusting base (34) which are coaxially disposed on the mandrel (20), and each of the clamping structures (40) is respectively connected to the clamping base (32) and the adjusting base (34); and
wherein when the locking structure (30) is gradually switched from the non-locking state to the locking state, a spacing distance between the clamping base (32) and the adjusting base (34) is gradually decreased, and a pivoting angle between each of the clamping structures (40) and the mandrel (20) is gradually decreased.

4. The clamping instrument according to claim 3, wherein the clamping base (32) is movably disposed on the mandrel (20) and capable of reciprocating in an axial direction of the mandrel (20); and the adjusting base (34) is fixedly disposed on the mandrel (20).

5. The clamping instrument according to claim 4, wherein the locking structure (30) further comprises a first fastener (36) and a second fastener (38) which are respectively disposed on the clamping base (32) and the adjusting base (34);
wherein the first fastener (36) and the second fastener (38) are configured to be buckled with each other to maintain the locking structure (30) to be in the locking state; and
wherein after the first fastener (36) and the second fastener (38) are buckled with each other, at least a portion of the mandrel (20) will retain in the clamping instrument (10), or the mandrel (20) will be completely withdrawn from the clamping instrument (10).

6. The clamping instrument according to claim 5, wherein each of the clamping structures (40) respectively comprises a main clamping arm (42), an auxiliary clamping arm (46), and an elastic arm (44);
the main clamping arm (42) and the auxiliary clamping arm (46) are pivotally connected to the clamping base (32) respectively, and two opposite ends of the elastic arm (44) are pivotally connected to the adjusting base (34) and the main clamping arm (42) respectively;
wherein when the locking structure (30) performs the switching action between the locking state and the non-locking state, the spacing distance between the clamping base (32) and the adjusting base (34) will be increased or decreased, so as to drive each of the main clamping arms (42) to pivot relative to the clamping base (32) via the elastic arm (44); and
when the locking structure (30) is maintained to be in the locking state, a continuous elastic pushing force will be applied to each of the main clamping arms (42) via each of the elastic arms (44) to urge each of the main clamping arms (42) to pivot towards a direction close to the auxiliary clamping arm (46) relative to the clamping base (32), such that a clamping angle formed between the main clamping arm (42) and the auxiliary clamping arm (46) is maintained to be minimum.

7. The clamping instrument according to claim 6, wherein when the locking structure (30) is in the locking state, the main clamping arm (42) will apply an opposite acting force to the elastic arm (44) according to a thickness of the target tissue, so as to elastically deform the elastic arm (44) to different degrees until an elastic pushing force of the elastic arm (44) is balanced with the opposite acting force of the main clamping arm (42).

8. The clamping instrument according to claim 7, wherein the elastic arm (44) comprises at least one of an arcuate bent part, an S-shaped bent part, and a Z-shaped bent part.

9. The clamping instrument according to claim 6, wherein each of the main clamping arms (42) is enable to be coaxially or non-coaxially pivoted to the clamping base (32).

10. The clamping instrument according to claim 6, wherein
the auxiliary clamping arm (46) is configured to be elastically deformed with a stress being applied, so as to pivot towards a direction away from the main clamping arm (42) relative to the clamping base (32), such that the clamping angle formed between the auxiliary clamping arm (46) and the main clamping arm (42) is increased; and
the auxiliary clamping arm (46) is configured to be elastically recovered without a stress being applied, so as to pivot towards a direction close to the main clamping arm (42) relative to the clamping base (32), such that the clamping angle formed between the auxiliary clamping arm (46) and the main clamping arm (42) is decreased.

11. The clamping instrument according to claim 10, wherein the auxiliary clamping arm (46) comprises a clamping part (462) and a pivoting part (464), and the clamping part (462) is pivoted to the clamping base (32) via the pivoting part (464); and
the pivoting part (464) is configured to be elastically deformed with a stress being applied and elastically recovered without a stress being applied, so as to allow the clamping part (462) to pivot relative to the clamping base (32).

12. The clamping instrument according to claim 10, wherein each of the auxiliary clamping arms (46) is of an integrated structure or a separate structure.
